# EUROPEAN PATENT APPLICATION

(11) **EP 4 717 701 A1**
(43) Date of publication of application: **01.04.2026**
(21) Application number: 24202489.1
(22) Date of filing: 25.09.2024
(51) Int. Cl.: C07K 1/22, C07K 1/36, C07K 19/00

(54) **METHOD FOR PURIFYING FC CONTAINING POLYPEPTIDES**

(71) Applicant: Formycon AG, 82152 Planegg (DE)
(72) Inventor: REITER, Alwin, 80337 München (DE)
(74) Representative: Maiwald GmbH

(57) **Abstract**

The invention relates to methods of purifying a polypeptide containing an Fc region comprising a Protein A chromatography step.

## Description

### Background

Therapeutic antibodies have become the predominant class of new drugs developed in recent years as they have exhibited outstanding efficacy and safety in the treatment of several major diseases including cancers, immune-related diseases, infectious disease and haematological disease. There has been significant progress in the global research and development of manufacturing processes of antibodies in the last decade.

Fc-based fusion proteins are composed of an immunoglobin Fc domain that is directly linked to another protein and are also of high therapeutic importance. The presence of the Fc domain markedly increases their plasma half-life, which prolongs therapeutic activity, owing to its interaction with the salvage neonatal Fc-receptor as well as to the slower renal clearance for larger sized molecules. The attached Fc domain also enables these molecules to interact with Fc-receptors (FcRs) found on immune cells, a feature that is particularly important for their use in oncological and anti-infectious therapies as well as in vaccines. Moreover, the Fc region allows for easy cost-effective purification by protein-G/A affinity chromatography during manufacture.

Protein A chromatography is a widely adopted affinity capture step for large-scale purification of antibodies and Fc fusion proteins. However, the low pH conditions typically required for Protein A elution can often lead to aggregation issues for these products especially when purifying antibodies and Fc fusion proteins having low isoelectric point, the so-called "acid-labile" proteins. Acid-labile proteins, therefore, pose a significant challenge to affinity capture, as traditional acid elution leads to protein aggregation or fragmentation.

Therefore, there is continued need for improved protein A chromatography conditions as well as further purification steps.

It is an object of the present invention to provide a protein A chromatography method that leads to improved recovery when purifying an Fc region containing polypeptide.

It is an object of the present invention to provide a protein A chromatography method that leads to prevention of aggregates formation when purifying an Fc region containing polypeptide.

It is further an object of the present invention to provide an improved viral inactivation method when purifying an Fc region containing polypeptide.

It is further an object of the present invention to provide an improved hydrophobic interaction chromatography method when purifying an Fc region containing polypeptide.

### Brief description of the invention

**The** invention provides a method of purifying a polypeptide containing an Fc region, the method comprising
- a Protein A chromatography step, wherein the Protein A chromatography step comprises an elution step with an elution buffer to obtain a Protein A eluate, wherein the elution buffer comprises arginine, and wherein the elution buffer has a pH of higher than 3.5,
wherein the Fc region is a variant of the human IgG1, IgG2, or IgG4 subclass and comprises at least one amino acid substitution selected from M252Y, S254T, and T256E according to EU numbering.

In some embodiments, the Fc region comprises amino acid substitutions M252Y, S254T, and T256E according to EU numbering.

In some embodiments, the elution buffer has a pH from 3.8 or higher.

In some embodiments, the elution buffer has a pH of 4.0 or higher such as from 4.2 to 4.5 such as 4.3.

In some embodiments, the elution buffer comprises 0.5 to 2.5 M L-arginine HCL, such as 1.0 to 2.5 M, 1.5 to 2.5 M, 1.8 to 2.3 M L-arginine HCL such as 2.0 M L-arginine HCL.

In some embodiments, the method comprises a virus inactivation step on the Protein A eluate comprising adding caprylic acid or a salt thereof.

In some embodiments, the method comprises a virus inactivation step on the Protein A eluate comprising the following sequential steps,
(i) pH adjustment comprising adjusting the pH to greater than 5.0 such as from 5.2 to 6.0 such as 5.6,
(ii) adding caprylic acid or a salt thereof.

In some embodiments, the method comprises adding sodium caprylate and the final concentration of sodium caprylate is from 10 to 30 mM.

In some embodiments, after the virus inactivation step, the method comprises one or more further purification steps.

In some embodiments, after the virus inactivation step, the method comprises the following sequential steps:
(i) a first UF/DF,
(ii) anion exchange chromatography preferably in bind-elute mode,
(iii) hydrophobic interaction chromatography preferably in flow-through mode,
(iv) viral filtration,
(v) a second UF/DF,
(vi) formulation,
(vii) drug substance storage.

In some embodiments, the hydrophobic interaction chromatography comprises a loading step comprising loading a load onto the hydrophobic interaction chromatography column, wherein the load comprises eluate obtained from the anion exchange chromatography comprising the polypeptide containing an Fc region and a loading buffer, wherein the loading buffer comprises zinc or a salt thereof.

In some embodiments, the hydrophobic interaction chromatography comprises an equilibration step with an equilibration buffer comprising zinc or a salt thereof before the loading step.

In some embodiments, the molar concentration of zinc or salt thereof in the equilibration buffer is two times or three times the molar concentration of the polypeptide containing an Fc region in the load.

In some embodiments, the concentration of zinc or salt thereof in the loading buffer results in a concentration of the zinc or salt thereof in the load that is two times or three times the molar concentration of the polypeptide containing an Fc region in the load.

In some embodiments, the concentration of zinc or salt thereof in the load is two times or three times the molar concentration of the polypeptide containing an Fc region in the load.

In some embodiments, the equilibration buffer, the loading buffer and/or the load comprises zinc chloride.

In some embodiments, recovery of the Fc region containing polypeptide after the Protein A chromatography step is higher and the percentage of high molecular weight aggregates is lower as compared to the same Fc region containing polypeptide not comprising at least one or all amino acid substitution (s) selected from M252Y, S254T, and T256E according to EU numbering and subjected to a Protein A chromatography step comprising an elution step with an elution buffer in which the elution buffer does not comprise arginine and has a pH of 3.5 or less.

In some embodiments, recovery of the Fc region containing polypeptide after the Protein A chromatography step is higher and the percentage of high molecular weight aggregates is lower as compared to the same Fc region containing polypeptide not comprising at least one or all amino acid substitution (s) selected from M252Y, S254T, and T256E according to EU numbering and subjected to a Protein A chromatography step comprising an elution step with an elution buffer in which the elution buffer does not comprise arginine and has a pH of 4.0 or less.

In some embodiments, a method for prevention of aggregates formation when purifying a polypeptide containing an Fc region of the human IgG1, IgG2, or IgG4 subclass is provided, the method comprising,
- providing a variant of the Fc region, wherein the variant comprises at least one or all amino acid substitutions selected from M252Y, S254T, and T256E according to EU numbering,
- a Protein A chromatography step, wherein the Protein A chromatography step comprises an elution step with an elution buffer, wherein the elution buffer comprises arginine, and wherein the elution buffer has a pH of higher than 3.5.

In some embodiments, a method for prevention of aggregates formation when purifying a polypeptide containing an Fc region of the human IgG1, IgG2, or IgG4 subclass is provided, the method comprising,
- providing a variant of the Fc region, wherein the variant comprises at least one or all amino acid substitutions selected from M252Y, S254T, and T256E according to EU numbering,
- a Protein A chromatography step, wherein the Protein A chromatography step comprises an elution step with an elution buffer, wherein the elution buffer comprises arginine, and wherein the elution buffer has a pH of 4.0 or higher.

In some embodiments, a method for prevention of aggregates formation when purifying a polypeptide containing an Fc region of the human IgG1, IgG2, or IgG4 subclass is provided, the method comprising,
- providing a variant of the Fc region, wherein the variant comprises at least one or all amino acid substitutions selected from M252Y, S254T, and T256E according to EU numbering,
- a Protein A chromatography step, wherein the Protein A chromatography step comprises an elution step with an elution buffer, wherein the elution buffer comprises arginine, wherein the elution buffer has a pH of higher than 3.5 such as 4.0 or higher, and wherein the elution buffer comprises 0.5 to 2.5 M arginine, such as 1.8 to 2.3 M arginine such as 2.0 M arginine.

In some embodiments, a method of viral inactivation of an eluate obtained from a Protein A chromatography step comprising an Fc region containing polypeptide, the method comprising
- eluting the Fc region containing polypeptide in the Protein A chromatography step using an elution buffer, wherein the elution buffer comprises arginine, and wherein the elution buffer has a pH of higher than 3.5 such as 4.0 or higher,
- adding sodium caprylate at a final concentration from 10 to 30 mM.

In some embodiments, the polypeptide is a fusion protein.

In some embodiments, the polypeptide has an isoelectric point of lower than 7 or lower than 6.

In some embodiments, the polypeptide is acid labile.

In some embodiments, the polypeptide is an ACE2-IgG1 or ACE2-IgG4 fusion protein.

### Definitions

The term "Fc region" herein is used to define a C-terminal region of an antibody heavy chain, including, for example, native sequence Fc regions, recombinant Fc regions, and variant Fc regions.

The term "YTE mutation" refers to the combination of mutations M252Y (Met252Tyr), S254T (Ser254Thr), and T256E (Thr256Glu) in the constant heavy chain region of IgG1 or IgG4 subclass, wherein the numbering is according to the EU numbering.

As used herein, the amino acid positions of all constant regions and domains of the heavy and light chain are numbered according to EU numbering as described in Edelman, G.M. et al., Proc. Natl. Acad. USA, 63, 78-85 (1969).

The term "variant" refers to any naturally occurring or engineered molecule comprising one or more nucleotide or amino acid mutations. For example, the term "Fc region is a variant of the human IgG1, IgG2, or IgG4 subclass" refers to an Fc region that differs from the IgG1, IgG2, or IgG4 subclass by one or more amino acid mutations.

The term "amino acid mutation" denotes the substitution of at least one existing amino acid residue with another different amino acid residue (= replacing amino acid residue). The replacing amino acid residue may be a "naturally occurring amino acid residue" and selected from the group consisting of alanine (three letter code: Ala, one letter code: A), arginine (Arg, R), asparagine (Asn, N), aspartic acid (asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V). The replacing amino acid residue may be a "non-naturally occurring amino acid residue". See e.g. US 6,586,207, WO 98/48032, WO 03/073238, US 2004/0214988, WO 2005/35727, WO 2005/74524, Chin, J.W., et al., J. Am. Chem. Soc. 124 (2002) 9026-9027; Chin, J.W. and Schultz, P.G., ChemBioChem 11 (2002) 1135-1137; and, Wang, L. and Schultz, P.G., Chem. Commun. (2002) 1-11.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and m, respectively.

The term "Fc-fusion polypeptide" denotes a fusion of a binding domain (e.g. an antigen binding domain such as a single chain antibody, or a polypeptide such as a ligand of a receptor, or a receptor of a ligand) with an antibody Fc-region that exhibits the desired target- effector function-, protein A- and FcRn-binding activity. In some embodiments, the domain is an enzymatically active domain.

The term "isoelectric point" refers to the pH at which a molecule carries no net electrical charge or is electrically neutral. The standard nomenclature to represent the isoelectric point is pH. However, pI is also used. Methods of calculating isoelectric point of proteins are known in the art. One widely used technique for this purpose is capillary Isoelectric Focusing (cIEF) technology. cIEF utilizes the principle of electrophoresis to separate and analyse proteins based on their isoelectric points. In this method, a capillary tube is filled with a pH gradient, typically created using a mixture of carrier ampholytes or immobilized pH gradient (IPG) strips. The protein sample is then introduced into the capillary tube and subjected to an electric field. As the electric current is applied, proteins migrate through the pH gradient until they reach a pH value matching their isoelectric point. At this point, the proteins cease to migrate further due to their neutrality, resulting in their sharp focusing at specific positions along the capillary. The separated protein bands can be visualized using various detection methods such as UV absorbance, fluorescence, or the cIEF instrument can be coupled to a mass spectrometer. cIEF technology offers high resolution, sensitivity, and automation, making it a valuable tool for precise determination and characterization of isoelectric points in complex protein mixtures. An exemplary method is provided in Goyon et al. 2017 Journal of Chromatography B, Volumes 1065-1066, Pages 119-128.

The term "acid labile" as used herein refers to an Fc region containing polypeptide that is prone to aggregate or denature under acidic conditions.

A "humanized" antibody refers to a chimeric antibody comprising amino acid residues from non-human highly variable repeats (HVRs) and amino acid residues from human framework regions (FRs). In certain embodiments, a humanized antibody will comprise substantially all of at least one, and typically two, variable domains, in which all or substantially all of the HVRs (e.g., the complementarity determining regions (CDRs)) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

A "human antibody" is one that possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "valent" as used within the current application denotes the presence of a specified number of binding sites in a (antibody) molecule. As such, the terms "bivalent", "tetravalent", and "hexavalent" denote the presence of two binding sites, four binding sites, and six binding sites, respectively, in a (antibody) molecule. The bispecific antibodies are "bivalent".

### Brief description of Figures

**Figure 1****:** Results of total recovery (%) of Fc region containing polypeptide, in the present case, ACE2-IgG under three different elution conditions (see Example 4).
**Figure 2****:** Results of total aggregates (HMW%) of Fc region containing polypeptide, in the present case, ACE2-IgG under three different elution conditions (see Example 4).
**Figure 3****:** Results of recovery (%) of Fc region containing polypeptide, in the present case, ACE2-IgG under three different elution conditions (see Example 4). The results show a synergistic effect of the combination of presence of arginine, pH higher than 3.5 and YTE mutation on total recovery.

### Detailed description of the invention

### Polypeptide containing an Fc region or Fc region containing a polypeptide

The present invention pertains to purifying a polypeptide containing an Fc region.

In some embodiments the polypeptide containing an Fc region to be purified has an isoelectric point of lower than 7. In some embodiments the polypeptide containing an Fc region to be purified has an isoelectric point of lower than 6.

In some embodiments the polypeptide containing an Fc region to be purified has an isoelectric point of lower than 7 as measured by capillary Isoelectric Focusing. In some embodiments the polypeptide containing an Fc region to be purified has an isoelectric point of lower than 6 as measured by capillary Isoelectric Focusing.

In some embodiments the polypeptide containing an Fc region to be purified is acid labile.

In some embodiments, the polypeptide containing an Fc region to be purified is an antibody.

In some embodiments, the polypeptide containing an Fc region to be purified is an IgG1, IgG2, or IgG4 antibody. In one embodiment the antibody is a monospecific antibody. In one embodiment the monospecific antibody is a monovalent monospecific antibody. In one embodiment the monospecific antibody is a bivalent monospecific antibody. In one embodiment the antibody is a bispecific antibody. In one embodiment the bispecific antibody is a bivalent bispecific antibody. In one embodiment the bispecific antibody is a tetravalent bispecific antibody. In one embodiment the antibody is a trispecific antibody. In one embodiment the trispecific antibody is a trivalent trispecific antibody. In one embodiment the trispecific antibody is a tetravalent trispecific antibody.

In some embodiments, the polypeptide containing an Fc region to be purified is a human antibody. In some embodiments, the polypeptide containing an Fc region to be purified is a humanized antibody.

In some embodiments, the polypeptide containing an Fc region to be purified is a fusion polypeptide. In some embodiments, the polypeptide containing an Fc region to be purified is an IgG1, IgG2, or IgG4 fusion protein.

In some embodiments, the polypeptide containing an Fc region to be purified is a human IgG1, IgG2, or IgG4 fusion protein. In some embodiments, the polypeptide containing an Fc region to be purified is a humanized IgG1, IgG2, or IgG4 fusion protein.

In some embodiments, the polypeptide containing an Fc region to be purified is an ACE2-IgG1, ACE2-IgG2, or ACE2-IgG4 fusion protein. In some embodiments, the polypeptide containing an Fc region to be purified is an ACE2-IgG1, or ACE2-IgG4 fusion protein. ACE2 (angiotensin converting enzyme 2) is a key metalloprotease of the renin-angiotensin system with a catalytic zinc ion in the centre (Donoghue et al. (2002) Circ. Res. 87: e1-e9 and Vickers at al. (2002) Biol. Chem. 277: 14838-43).

In some embodiments, ACE2 has an amino acid sequence according to SEQ ID NO:1.

In some embodiments, wild type IgG1 has an amino acid sequence according to SEQ ID NO: 2. In some embodiments, wild type IgG4 has an amino acid sequence according to SEQ ID NO: 3.

In some embodiments, the polypeptide containing an Fc region to be purified comprises an Fc region that is a variant of the human IgG1, IgG2, or IgG4 subclass. In some embodiments, the polypeptide containing an Fc region to be purified comprises an Fc region that is a variant of the human IgG1, IgG2, or IgG4 subclass and comprises at least one amino acid substitution selected from M252Y, S254T, and T256E according to EU numbering.

In some embodiments, the polypeptide containing an Fc region to be purified comprises an Fc region that is a variant of the human IgG1, IgG2, or IgG4 subclass and comprises amino acid substitutions M252Y, S254T, and T256E according to EU numbering.

In some embodiments, IgG1 has an amino acid sequence according to SEQ ID NO: 4. In some embodiments, IgG4 has an amino acid sequence according to SEQ ID NO: 5.

In some embodiments, ACE2-IgG has an amino acid sequence according to SEQ ID NO:6 to 7.

In some embodiments, ACE2-IgG1 has an amino acid sequence according to SEQ ID NO:6. In some embodiments, ACE2 and IgG1 are linked by a linker. In some embodiments, the linker is the hinge region of human IgG1. In some embodiments, the linker amino acid sequence is according to SEQ ID No. 8.

In some embodiments, ACE2-IgG4 has an amino acid sequence according to SEQ ID NO:7. In some embodiments, ACE2 and IgG4 are linked by a linker. In some embodiments, the linker is the hinge region of human IgG4. In some embodiments, the linker amino acid sequence is according to SEQ ID No. 9.

### Method of purifying a polypeptide containing an Fc region - Protein A chromatography step

The present invention provides a method of purifying a polypeptide containing an Fc region as described in the previous section.

In some embodiments, the polypeptide containing an Fc region is a variant of the human IgG1, IgG2, or IgG4 subclass. In some embodiments, the polypeptide containing an Fc region is a variant of the human IgG1, IgG2, or IgG4 subclass comprising at least one amino acid substitution selected from M252Y, S254T, and T256E according to EU numbering. In some embodiments, the polypeptide containing an Fc region is a variant of the human IgG1, IgG2, or IgG4 subclass comprising amino acid substitutions M252Y, S254T, and T256E according to EU numbering.

In one embodiment, a method of purifying a polypeptide containing an Fc region comprises a Protein A chromatography step. In one embodiment, a method of purifying a polypeptide containing an Fc region comprises a Protein A chromatography step comprising an elution step with an elution buffer. In one embodiment, the elution buffer comprises arginine or a salt thereof. In one embodiment the elution buffer comprises L-arginine HCL. In one embodiment, the elution buffer has a pH of higher than 3.5.

In some embodiments, the pH of the elution buffer is higher than 3.5. In some embodiments, the pH of the elution buffer is 3.8 or higher. In some embodiments, the pH of the elution buffer is 4.0 or higher. In some embodiments, the pH of the elution buffer is from 4.2 to 4.5 such as 4.3.

In some embodiments, the elution buffer comprises 0.5 M to 2.5 M L-arginine HCL. In some embodiments, the elution buffer comprises 1.5 M to 2.5 M L-arginine HCL. In some embodiments, the elution buffer comprises 1.8 M to 2.3 M L-arginine HCL such as 2.0 M L-arginine HCL.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of higher than 3.5, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 3.5, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 3.5, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 3.5, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of 3.8 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of 3.8 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of 3.8 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of 3.8 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of 3.8 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of 3.8 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of 3.8 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of 3.8 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of 4.0 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of 4.0 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of 4.0 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of 4.0 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of 4.0 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of 4.0 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of 4.0 or higher, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of 4.0 or higher, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of 4.2 to 4.5 such as 4.3, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of 4.2 to 4.5 such as 4.3, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of 4.2 to 4.5 such as 4.3, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of 4.2 to 4.5 such as 4.3, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of 4.2 to 4.5 such as 4.3, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of 4.2 to 4.5 such as 4.3, wherein the Fc region is a variant of the human IgG1, IgG2, or IgG4 subclass and comprises amino acid substitutions M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of 4.2 to 4.5 such as 4.3, wherein the Fc region is a variant of the human IgG1, IgG2, or IgG4 subclass and comprises at least one amino acid substitution selected from M252Y, S254T, and T256E according to EU numbering.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of 4.2 to 4.5 such as 4.3 wherein the Fc region is a variant of the human IgG1, IgG2, or IgG4 subclass and comprises amino acid substitutions M252Y, S254T, and T256E according to EU numbering.

In some embodiments disclosed above, the elution buffer further comprises acetate. In some embodiments disclosed above, the elution buffer further comprises sodium chloride. In some embodiments disclosed above, the elution buffer further comprises acetate and sodium chloride.

In some embodiments disclosed above, the elution buffer further comprises 10 to 50 mM acetate. In some embodiments disclosed above, the elution buffer further comprises 20 to 50 mM acetate such as 40 mM acetate. In some embodiments disclosed above, the elution buffer further comprises 20 to 70 mM sodium chloride. In some embodiments disclosed above, the elution buffer further comprises 30 to 60 mM sodium chloride such as 50 mM sodium chloride. In some embodiments disclosed above, the elution buffer further comprises 10 to 50 mM acetate and 20 to 70 mM sodium chloride. In some embodiments disclosed above, the elution buffer further comprises 20 to 50 mM acetate such as 40 mM acetate and 30 to 60 mM such as 50 mM sodium chloride.

In some embodiments, the Protein A chromatography comprises an equilibration step with an equilibration buffer. In some embodiments, the equilibration step comprises equilibrating the Protein A chromatography column with an equilibration buffer. In some embodiments, the equilibration buffer comprises MES and salt preferably sodium chloride at pH from 5.5 to 6.7 such as 6.5. In some embodiments, the equilibration buffer comprises MES at a concentration from 20 to 100 mM and sodium chloride at a concentration from 50 to 200 mM. In some embodiments, the equilibration buffer comprises MES at a concentration from 30 to 80 mM such as 40 mM and sodium chloride at a concentration from 80 to 180 mM such as 150 mM.

In some embodiments, the Protein A chromatography comprises at least one intermediate wash step. In some embodiments, the Protein A chromatography comprises at least one intermediate wash step with a wash buffer. In some embodiments, the Protein A chromatography comprises at least two intermediate wash steps with a wash buffer. In some embodiments, the Protein A chromatography comprises at least three intermediate wash steps with a wash buffer. In some embodiments, the Protein A chromatography comprises at least four intermediate wash steps with a wash buffer and an equilibration buffer.

In some embodiments, the wash buffer comprises a salt preferably sodium chloride and a buffer such as MES or acetate buffer. In some embodiments, the wash buffer comprises MES or acetate at a concentration from 20 to 100 mM and salt preferably sodium chloride at a concentration from 20 to 1500 mM at pH 5.5 to 6.7 such as 5.5 to 6.5.

In some embodiments, the wash buffer comprises a salt preferably sodium chloride and a buffer such as MES buffer. In some embodiments, the wash buffer comprises MES at a concentration from 20 to 100 mM and salt preferably sodium chloride at a concentration from 20 to 5000 mM. In some embodiments, the wash buffer comprises MES at a concentration from 30 to 80 mM such as 40 mM and salt preferably sodium chloride at a concentration from 800 to 1500 mM such as 1000 mM. In some embodiments, the pH of the wash buffer is from 5.5 to 6.7 such as 6.5.

In some embodiments, the wash buffer comprises a salt preferably sodium chloride and a buffer such as MES buffer. In some embodiments, the wash buffer comprises MES at a concentration from 20 to 100 mM and salt preferably sodium chloride at a concentration from 20 to 70 mM. In some embodiments, the wash buffer comprises MES at a concentration from 30 to 80 mM such as 40 mM and salt preferably sodium chloride at a concentration from 20 to 70 mM such as 50 mM. In some embodiments, the pH of the wash buffer is from 5.5 to 6.7 such as 6.5.

In some embodiments, the wash buffer comprises a salt preferably sodium chloride and a buffer such as sodium acetate buffer. In some embodiments, the wash buffer comprises sodium acetate at a concentration from 20 to 100 mM and salt preferably sodium chloride at a concentration from 20 to 70 mM. In some embodiments, the wash buffer comprises sodium acetate at a concentration from 30 to 80 mM such as 40 mM and salt preferably sodium chloride at a concentration from 20 to 70 mM such as 50 mM. In some embodiments, the pH of the wash buffer is from 5.0 to 6.0 such as 5.6.

In some embodiments, the Protein A chromatography comprises at least three intermediate wash steps with the same or different wash buffer.

In some embodiments, the first wash after wash with equilibration buffer is carried out with a first wash buffer comprising a salt preferably sodium chloride and a buffer such as MES buffer. In some embodiments, the first wash buffer comprises MES at a concentration from 20 to 100 mM and salt preferably sodium chloride at a concentration from 20 to 5000 mM. In some embodiments, the first wash buffer comprises MES at a concentration from 30 to 80 mM such as 40 mM and salt preferably sodium chloride at a concentration from 800 to 1500 mM such as 1000 mM. In some embodiments, the pH of the first wash buffer is from 5.5 to 6.7 such as 6.5.

In some embodiments, the second wash after wash with equilibration buffer is carried out with a second wash buffer comprising a salt preferably sodium chloride and a buffer such as MES buffer. In some embodiments, the second wash buffer comprises MES at a concentration from 20 to 100 mM and salt preferably sodium chloride at a concentration from 20 to 70 mM. In some embodiments, the second wash buffer comprises MES at a concentration from 30 to 80 mM such as 40 mM and salt preferably sodium chloride at a concentration from 20 to 70 mM such as 50 mM. In some embodiments, the pH of the second wash buffer is from 5.5 to 6.7 such as 6.5.

In some embodiments, the third wash after wash with equilibration buffer is carried out with a third wash buffer comprising a salt preferably sodium chloride and a buffer such as sodium acetate buffer. In some embodiments, the third wash buffer comprises sodium acetate at a concentration from 20 to 100 mM and salt preferably sodium chloride at a concentration from 20 to 70 mM. In some embodiments, the third wash buffer comprises sodium acetate at a concentration from 30 to 80 mM such as 40 mM and salt preferably sodium chloride at a concentration from 20 to 70 mM such as 50 mM. In some embodiments, the pH of the third wash buffer is from 5.0 to 6.0 such as 5.6.

At the end of the Protein A chromatography step described herein above, an eluate comprising the Fc region containing polypeptide is obtained. In some embodiments, the Protein A eluate comprising the Fc region containing polypeptide is subjected to further purification steps.

### Method of purifying a polypeptide containing an Fc region - Viral inactivation

In some embodiments, a method of viral inactivation of a Protein A eluate comprising an Fc region containing polypeptide is provided.

In some embodiments, a method of viral inactivation of the Protein A eluate comprising the Fc region containing polypeptide as described in the previous section is provided. In some embodiments, a method of viral inactivation of the Protein A eluate comprising the Fc region containing polypeptide and arginine as described in the previous section is provided.

In some embodiments, the method of viral inactivation comprises adding caprylic acid or a salt thereof. In some embodiments, the final concentration of caprylic acid or a salt thereof is from 10 to 30 mM. In some embodiments, the final concentration of caprylic acid or a salt thereof is from 15 to 30 mM such as 25 mM.

In some embodiments, the method of viral inactivation comprises adding sodium caprylate. In some embodiments, the final concentration of sodium caprylate is from 10 to 30 mM. In some embodiments, the final concentration of sodium caprylate is from 15 to 30 mM such as 25 mM.

In some embodiments, the viral inactivation step comprises pH adjustment of the Protein A eluate. In some embodiments, the pH adjustment step comprises adjusting the pH to greater than 5.0. In some embodiments, the pH adjustment step comprises adjusting the pH from 5.2 to 6.0. In some embodiments, the pH adjustment step comprises adjusting the pH from 5.5 to 6.0. In some embodiments, the pH adjustment step comprises adjusting the pH from 5.5 to 5.7 such as 5.6.

In some embodiments, the viral inactivation step comprises pH adjustment of the Protein A eluate followed by adding caprylic acid or a salt thereof. In some embodiments, the pH adjustment step comprises adjusting the pH to greater than 5.0. In some embodiments, the pH adjustment step comprises adjusting the pH to greater than 5.2 to 6.0. In some embodiments, the pH adjustment step comprises adjusting the pH to greater than 5.5 to 6.0 such as 5.6. In some embodiments, the final concentration of caprylic acid or a salt thereof is from 10 to 30 mM. In some embodiments, sodium caprylate is added. In some embodiments, the final concentration of sodium caprylate is from 10 to 30 mM such as 25 mM.

In some embodiments, the viral inactivation step comprises the following sequential steps: (i) pH adjustment comprising adjusting the pH to greater than 5.0 such as from 5.2 to 6.0 such as 5.6, (ii) adding caprylic acid or a salt thereof. In some embodiments, the viral inactivation step comprises the following sequential steps: (i) pH adjustment comprising adjusting the pH to greater than 5.0 such as from 5.5 to 6.0 such as 5.6, (ii) adding caprylic acid or a salt thereof. In some embodiments, the viral inactivation step comprises the following sequential steps: (i) pH adjustment comprising adjusting the pH to greater than 5.0 such as from 5.5 to 5.7 such as 5.6, (ii) adding caprylic acid or a salt thereof.

In some embodiments, the viral inactivation step comprises the following sequential steps: (i) pH adjustment comprising adjusting the pH to greater than 5.0 such as from 5.2 to 6.0 such as 5.6, (ii) adding caprylic acid or a salt thereof at a final concentration from 10 to 30 mM such as 25 mM. In some embodiments, the viral inactivation step comprises the following sequential steps: (i) pH adjustment comprising adjusting the pH to greater than 5.0 such as from 5.5 to 6.0 such as 5.6, (ii) adding caprylic acid or a salt thereof at a final concentration from 10 to 30 mM such as 25 mM. In some embodiments, the viral inactivation step comprises the following sequential steps: (i) pH adjustment comprising adjusting the pH to greater than 5.0 such as from 5.5 to 5.7 such as 5.6, (ii) adding caprylic acid or a salt thereof at a final concentration from 10 to 30 mM such as 25 mM.

In some embodiments, the viral inactivation step comprises the following sequential steps: (i) pH adjustment comprising adjusting the pH to greater than 5.0 such as from 5.2 to 6.0 such as 5.6, (ii) adding sodium caprylate. In some embodiments, the viral inactivation step comprises the following sequential steps: (i) pH adjustment comprising adjusting the pH to greater than 5.0 such as from 5.5 to 6.0 such as 5.6, (ii) adding sodium caprylate. In some embodiments, the viral inactivation step comprises the following sequential steps: (i) pH adjustment comprising adjusting the pH to greater than 5.0 such as from 5.5 to 5.7 such as 5.6, (ii) adding sodium caprylate.

In some embodiments, the viral inactivation step comprises the following sequential steps: (i) pH adjustment comprising adjusting the pH to greater than 5.0 such as from 5.2 to 6.0 such as 5.6, (ii) adding sodium caprylate at a final concentration from 10 to 30 mM such as 25 mM. In some embodiments, the viral inactivation step comprises the following sequential steps: (i) pH adjustment comprising adjusting the pH to greater than 5.0 such as from 5.5 to 6.0 such as 5.6, (ii) adding sodium caprylate at a final concentration from 10 to 30 mM such as 25 mM. In some embodiments, the viral inactivation step comprises the following sequential steps: (i) pH adjustment comprising adjusting the pH to greater than 5.0 such as from 5.5 to 5.7 such as 5.6, (ii) adding sodium caprylate at a final concentration from 10 to 30 mM such as 25 mM.

In some embodiments, the pH adjustment step is conducted with one or more pH adjustment buffers. In some embodiments, the pH adjustment step is conducted with two pH adjustment buffers. In some embodiments, the two pH adjustment buffers are the same. In some embodiments, the two pH adjustment buffers are different. In some embodiments, the pH adjustment step is conducted with three pH adjustment buffers. In some embodiments, the three pH adjustment buffers are the same. In some embodiments, the three pH adjustment buffers are different.

In some embodiments, the pH adjustment buffer is an MES buffer. In some embodiments, the MES buffer comprises MES hydrate. In some embodiments, the MES hydrate is at a concentration from 100 to 300 mM. In some embodiments, the MES hydrate is at a concentration from 150 to 250 mM such as 200 mM. In some embodiments, the MES buffer further comprises a salt preferably sodium chloride. In some embodiments, the salt preferably sodium chloride is at a concentration from 20 to 70 mM such as 50 mM. In some embodiments, the pH of the MES buffer is from 6.0 to 6.7, such as 6.2

In some embodiments, the pH adjustment buffer is an acetate buffer. In some embodiments, the acetate buffer comprises acetic acid. In some embodiments, acetic acid is at a concentration from 200 to 600 mM. In some embodiments, acetic acid is at a concentration from 300 to 600 mM such as 500 mM. In some embodiments, the acetate buffer further comprises a salt preferably sodium chloride. In some embodiments, the salt preferably sodium chloride is at a concentration from 20 to 70 mM such as 50 mM.

In some embodiments, the pH adjustment buffer is a Tris buffer. In some embodiments, Tris is at a concentration from 500 mM to 1.5 M. In some embodiments, Tris is at a concentration from 800 mM to 1.2 M such as 1.0 M. In some embodiments, the pH of the Tris buffer is from 7.0 to 9.2, such as 9.0. In some embodiments, the Tris buffer further comprises a salt preferably sodium chloride. In some embodiments, the salt preferably sodium chloride is at a concentration from 20 to 70 mM such as 50 mM.

In some embodiments, the pH adjustment step is conducted with one buffer, which is the MES buffer. In some embodiments, the pH adjustment step is conducted with two buffers, wherein the first buffer is the MES buffer, and the second buffer is the acetate buffer. In some embodiments, the pH adjustment step is conducted with three buffers, wherein the first buffer is the MES buffer, the second buffer is the acetate buffer, and the third buffer is a Tris buffer.

In some embodiments, the viral inactivation step as described above is followed by an incubation step. In some embodiments, the incubation is from 30 to 120 minutes. In some embodiments, the incubation is from 30 to 90 minutes. In some embodiments, the incubation is from 60 to 90 minutes such as 60 to 80 minutes.

In some embodiments, the method of viral inactivation as described here restricts formation of aggregates at the viral inactivation step. In some embodiments, the method of viral inactivation as described here restricts formation of aggregates as measured by size exclusion chromatography at the viral inactivation step. In some embodiments, the method of viral inactivation as described here restricts formation of aggregates at the viral inactivation step to less than 2-5% such as less than 1% or less than 0.5%. In some embodiments, the method of viral inactivation as described here restricts formation of aggregates at the viral inactivation step to less than 2-5% such as less than 1% or less than 0.5% as measured by size exclusion chromatography.

In some embodiments, the viral inactivation step as described here in combination with the protein A chromatography step described in the previous section restricts formation of aggregates. In some embodiments, the viral inactivation step as described here in combination with the protein A chromatography step described in the previous section restricts formation of aggregates as measured by size exclusion chromatography. In some embodiments, the viral inactivation step as described here in combination with the protein A chromatography step described in the previous section provides improved recovery. In some embodiments, the viral inactivation step as described here in combination with the protein A chromatography step described in the previous section provides improved recovery.

At the end of the virus inactivation step described herein above, a virus inactivated composition comprising the Fc region containing polypeptide is obtained.

### Method of purifying a polypeptide containing an Fc region - ultrafiltration/diafiltration (UF/DF)

In some embodiments, a method of ultrafiltration/diafiltration on a composition comprising an Fc region containing polypeptide is provided. According to an embodiment of the invention, the ultrafiltration step can be a direct flow filtration (DFF) or tangential flow filtration step (TFF). Direct flow filtration (DFF) is applied when all the fluid to be filtered is driven, due to a supply pressure, in a direction perpendicular to a filtering surface. Contaminants or soluble proteins are captured within the filtration media or build up on the surface, causing the differential pressure across the filter to rise as it blocks over the duration of the filtration process. The filtrate exits the filter on the downstream side. Once a certain differential pressure has been reached, after which the fluid flow rate decreases and/or the filter reaches its terminal differential pressure, filtration is stopped, and the filter is either discarded or may sometimes be regenerated for re-use. On the other hand, Tangential Flow Filtration (TFF) is a process where the feed stream flows parallel to the membrane face. Applied pressure causes one portion of the flow stream to pass through the membrane (filtrate) while the remainder (retentate) is recirculated back to the feed reservoir. Thus, in TFF the majority of the feed travels tangentially across the surface of the filter and not into the filter.

In some embodiments, a method of ultrafiltration/diafiltration on the virus inactivated composition comprising the Fc region containing polypeptide as described in the previous section is provided.

In some embodiments, after the viral inactivation step, the virus inactivated composition comprising the Fc region containing polypeptide is optionally subjected to ultrafiltration/diafiltration.

In some embodiments, after the viral inactivation step, the virus inactivated composition comprising the Fc region containing polypeptide is subjected to a pH adjustment step immediately preceding UF/DF, wherein the target pH is from 7.0 to 7.7, from 7.4 to 7.6. In some embodiments, after the viral inactivation step, the virus inactivated composition comprising the Fc region containing polypeptide is subjected to a pH adjustment step immediately preceding UF/DF with a buffer comprising Tris and salt preferably sodium chloride. In some embodiments, Tris is at a concentration from 500 mM to 1.5 M and salt preferably sodium chloride is at a concentration from 20 to 70 mM such as 50 mM. In some embodiments, Tris is at a concentration from 800 mM to 1.2 M such as 1.0 M Tris and salt preferably sodium chloride is at a concentration from 20 to 70 mM such as 50 mM. In some embodiments, the pH of the buffer is from 7.0 to 9.2, such as 9.0.

In some embodiments, after the pH adjustment step, a virus inactivated and pH adjusted composition comprising the Fc region containing polypeptide is obtained.

In some embodiments, the virus inactivated composition comprising the Fc region containing polypeptide is subjected to at least one filtration step. In some embodiments, the virus inactivated and pH adjusted composition comprising the Fc region containing polypeptide is subjected to at least one filtration step. In some embodiments, the virus inactivated composition comprising the Fc region containing polypeptide is subjected to a UF/DF step. In some embodiments, the virus inactivated and pH adjusted composition comprising the Fc region containing polypeptide is subjected to a UF/DF step.

In some embodiments, the UF/DF step comprises concentrating the virus inactivated composition comprising the Fc region containing polypeptide to less than 20 g/L. In some embodiments, the UF/DF step comprises concentrating the virus inactivated composition comprising the Fc region containing polypeptide to less than 18 g/L. In some embodiments, the UF/DF step comprises concentrating the virus inactivated composition comprising the Fc region containing polypeptide to less than 15 g/L. In some embodiments, the UF/DF step comprises concentrating the virus inactivated composition comprising the Fc region containing polypeptide to 8 to 15 g/L. In some embodiments, the UF/DF step comprises concentrating the virus inactivated composition comprising the Fc region containing polypeptide to 10 to 15 g/L.

In some embodiments, the UF/DF step comprises concentrating the virus inactivated and pH adjusted composition comprising the Fc region containing polypeptide to less than 20 g/L. In some embodiments, the UF/DF step comprises concentrating the virus inactivated and pH adjusted composition comprising the Fc region containing polypeptide to less than 18 g/L. In some embodiments, the UF/DF step comprises concentrating the virus inactivated and pH adjusted composition comprising the Fc region containing polypeptide to less than 15 g/L. In some embodiments, the UF/DF step comprises concentrating the virus inactivated and pH adjusted composition comprising the Fc region containing polypeptide to 8 to 15 g/L. In some embodiments, the UF/DF step comprises concentrating the virus inactivated and pH adjusted composition comprising the Fc region containing polypeptide to 10 to 15 g/L.

In some embodiments, the UF/DF step comprises exchanging the buffer to a buffer comprising Tris and salt preferably sodium chloride. In some embodiments, the UF/DF step comprises exchanging the buffer to a buffer comprising Tris at a concentration from 10 to 100 mM and salt preferably sodium chloride at a concentration from 20 to 70 mM. In some embodiments, the UF/DF step comprises exchanging the buffer to a buffer comprising Tris at a concentration from 30 to 80 mM and salt preferably sodium chloride at a concentration from 20 to 70 mM such as 50 mM. In some embodiments, the pH of the buffer is from 7.0 to 9.2 such as 7.5.

At the end of the ultrafiltration/diafiltration step described herein above, a retentate comprising the Fc region containing polypeptide is obtained.

Method of purifying a polypeptide containing an Fc region - anion exchange chromatography

In some embodiments, a method of anion exchange chromatography on a composition comprising an Fc region containing polypeptide is provided.

In some embodiments, a method of anion exchange chromatography on the retentate comprising the Fc region containing polypeptide as described in the previous section is provided.

In some embodiments, after the ultrafiltration/diafiltration step, the retentate comprising the Fc region containing polypeptide is optionally subjected to an anion exchange chromatography step.

In some embodiments, anion exchange chromatography is conducted in bind-elute mode. In some embodiments, anion exchange chromatography is conducted in flow-through mode.

Commonly used anion exchange functional groups are Q-resin, a quaternary amine, and DEAE resin (diethylaminoethyl). However, in general the anion exchange chromatography step can be performed with all common commercially available anion exchange resins or membranes. Typical strong anion exchange groups that can be used for the purpose of the invention comprise functional groups such as: quaternary aminoethyl (QAE) moieties, primary amine (PA), quaternary ammonium (Q) moieties and trimethylammoniumethyl (TMAE) groups. Resins having quaternary aminoethyl (QAE) moieties include, e.g., Toyopearl QAE (available from Tosoh Bioscience, Germany), Selectacel QAE (a quaternary aminoethyl derivative of cellulose, available from Polysciences Inc., Pennsylvania USA) and others. Resins having quaternary ammonium (Q) moieties include, e.g., Sartobind^{®} Q (available from Sartorius, Germany), Mustang^{®} Q Acrodisc (available from Pall, Germany), Q Sepharose XL, Q Sepharose FF, Q Sepharose HP, Resource Q, Capto Q (available from Cytivia, USA), Macro Prep High Q (Bio-Rad, California, USA), Toyopearl Super Q (available from Tosoh Bioscience, Germany) and UNOsphere Q (available from Bio-Rad, California, USA). Resins having trimethylammoniumethyl (TMAE) groups include, e.g., Fractogel EMD TMAE (available from Merck, Germany). Resins having primary amine groups include Sartobind STIC primary amine resins (available from Sartorius, Germany).

In some embodiments, anion exchange chromatography comprises an equilibration step with an equilibration buffer. In some embodiments, the equilibration step comprises equilibrating the anion exchange chromatography column with an equilibration buffer. In some embodiments, the equilibration buffer comprises Tris and sodium chloride. In some embodiments, the equilibration buffer comprises Tris at a concentration from 10 to 100 mM and sodium chloride at a concentration from 20 to 70 mM. In some embodiments, the equilibration buffer comprises Tris at a concentration from 30 to 80 mM such as 50 mM and sodium chloride at a concentration from 20 to 70 mM such as 50 mM. In some embodiments, the pH of the equilibration buffer is from 7.0 to 9.2 such as 7.5.

In some embodiments, anion exchange chromatography comprises a loading step. In some embodiments, the loading step comprises loading the retentate from the UF/DF step as described in the previous section onto the anion exchange chromatography column.

In some embodiments, anion exchange chromatography comprises at least one intermediate wash step. In some embodiments, anion exchange chromatography comprises at least one intermediate wash step with a wash buffer. In some embodiments, the wash buffer comprises Tris and salt preferably sodium chloride. In some embodiments, the wash buffer comprises Tris at a concentration from 10 to 100 mM and salt preferably sodium chloride at a concentration from 20 to 70 mM. In some embodiments, the wash buffer comprises Tris at a concentration from 30 to 80 mM such as 50 mM and salt preferably sodium chloride at a concentration from 20 to 70 mM such as 50 mM. In some embodiments, the pH of the wash buffer is from 7.0 to 9.2 such as 7.5.

In some embodiments, anion exchange chromatography is conducted in flow-through mode and comprises a step of collection of the flow-through. In some embodiments, anion exchange chromatography is conducted in bind-elute mode and comprises an elution step with one or more elution buffers.

In some embodiments, when two elution buffers are used, the elution is a gradient elution. In some embodiments, the elution buffer comprises Tris and salt. In some embodiments, anion exchange chromatography comprises an elution step with two elution buffers varying in salt concentration. In some embodiment, the second elution buffer has 10 times more concentration of salt as compared to the concentration of salt in the first elution buffer. In some embodiments, anion exchange chromatography comprises an elution step with two elution buffers varying in pH. In some embodiments, the elution buffer comprises Tris at a concentration from 10 to 100 mM and salt preferably sodium chloride at a concentration from 20 to 700 mM.

At the end of the anion exchange chromatography step described herein above, an anion exchange chromatography eluate or flow-through comprising the Fc region containing polypeptide is obtained.

### Method of purifying a polypeptide containing an Fc region - Hydrophobic interaction chromatography

In some embodiments, a method of hydrophobic interaction chromatography on a composition comprising an Fc region containing polypeptide is provided.

In some embodiments, a method of hydrophobic interaction chromatography on the anion exchange chromatography eluate or flow-through comprising the Fc region containing polypeptide as described in the previous section is provided.

In some embodiments, after the anion exchange chromatography step, the eluate or flow-through comprising the Fc region containing polypeptide is optionally subjected to a hydrophobic interaction chromatography step.

In some embodiments, the hydrophobic interaction chromatography is conducted in bind-elute mode. In some embodiments, the hydrophobic interaction chromatography is conducted in flow-through mode.

In some embodiments, the hydrophobic interaction chromatography comprises a loading step comprising loading a load onto the hydrophobic chromatography column. In some embodiments, the load comprises the eluate or flow-through obtained from the anion exchange chromatography comprising the Fc region containing polypeptide and a loading buffer. In some embodiments, the load comprises a composition comprising an Fc region containing polypeptide and a loading buffer.

In some embodiments, the loading buffer comprises zinc or a salt thereof preferably zinc chloride. In some embodiments, the concentration of zinc or salt thereof preferably zinc chloride in the loading buffer results in a concentration of the zinc or salt thereof preferably zinc chloride in the load that is equal to, two times, three times or four times the molar concentration of the polypeptide containing an Fc region in the load.

In some embodiments, zinc or a salt thereof preferably zinc chloride is added to the loading buffer that results in a concentration of the zinc or salt thereof preferably zinc chloride in the load that is equal to, two times, three times or four times the molar concentration of the polypeptide containing an Fc region in the load.

In some embodiments, the concentration of zinc or salt thereof preferably zinc chloride in the load is equal to, two times, three times or four times the molar concentration of the polypeptide containing an Fc region in the load.

In some embodiments, the hydrophobic interaction chromatography comprises an equilibration step with an equilibration buffer comprising zinc or a salt thereof before the loading step. In some embodiments, the hydrophobic interaction chromatography comprises an equilibration step with an equilibration buffer. In some embodiments, the equilibration buffer comprises zinc or a salt thereof. In some embodiments, zinc or a salt thereof is added to the equilibration buffer. In some embodiments, the concentration of zinc or salt thereof preferably zinc chloride in the equilibration buffer is equal to, two times, three times or four times the molar concentration of the polypeptide containing an Fc region in the load.

In some embodiments, the hydrophobic interaction chromatography comprises an equilibration step with an equilibration buffer. In some embodiments, the equilibration buffer comprises ammonium sulphate. In some embodiments, ammonium sulphate is at a concentration from 300 to 800 mM. In some embodiments, ammonium sulphate is at a concentration from 400 to 700 mM such as 660 mM or 560 mM. In some embodiments, the equilibration buffer further comprises Tris at a concentration from 10 to 100 mM. In some embodiments, the equilibration buffer further comprises Tris at a concentration from 30 to 80 mM such as 50 mM. In some embodiments, the pH of the equilibration buffer is from 7.0 to 9.2 such as 7.5.

In some embodiments, the hydrophobic interaction chromatography comprises an equilibration step with an equilibration buffer. In some embodiments, the equilibration buffer comprises Tris and salt preferably sodium chloride. In some embodiments, Tris is at a concentration from 20 to 80 mM such as 50 mM. In some embodiments, salt preferably sodium chloride is at a concentration from 80 to 200 mM such as 150 mM. In some embodiments, the pH of the equilibration buffer is from 7.0 to 9.2 such as 7.5.

In some embodiments, the equilibration buffer further comprises zinc or a salt thereof. In some embodiments, zinc or a salt thereof is added to the equilibration buffer. In some embodiments, the concentration of zinc or salt thereof preferably zinc chloride in the equilibration buffer is equal to, two times, three times or four times the molar concentration of the polypeptide containing an Fc region in the load.

In some embodiments, adding zinc or salt thereof in the equilibration buffer results in improved stability of the Fc region containing polypeptide such as ACE2-Fc. In some embodiments, adding zinc or salt thereof in the equilibration buffer results in improved stability of the Fc region containing polypeptide such as ACE2-Fc in terms of acidic variants as measured by anion exchange chromatography. In some embodiments, adding zinc or salt thereof in the equilibration buffer results in improved stability of the Fc region containing polypeptide such as ACE2-Fc in terms of basic variants as measured by anion exchange chromatography. In some embodiments, adding zinc or salt thereof in the equilibration buffer prevents formation of aggregates of the Fc region containing polypeptide such as ACE2-Fc as measured by size exclusion chromatography.

In some embodiments, adding zinc or salt thereof in the loading buffer results in improved stability of the Fc region containing polypeptide such as ACE2-Fc. In some embodiments, adding zinc or salt thereof in the loading buffer results in improved stability of the Fc region containing polypeptide such as ACE2-Fc in terms of acidic variants as measured by anion exchange chromatography. In some embodiments, adding zinc or salt thereof in the loading buffer results in improved stability of the Fc region containing polypeptide such as ACE2-Fc in terms of basic variants as measured by anion exchange chromatography. In some embodiments, adding zinc or salt thereof in the loading buffer prevents formation of aggregates of the Fc region containing polypeptide such as ACE2-Fc as measured by size exclusion chromatography.

In some embodiments, the loading buffer comprises ammonium sulphate. In some embodiments, ammonium sulphate is at a final concentration from 300 to 800 mM in the load. In some embodiments, ammonium sulphate is at a final concentration from 400 to 700 mM such as 660 mM or 560 mM in the load. In some embodiments, the loading buffer further comprises Tris. In some embodiments, Tris is at a final concentration from 10 to 100 mM in the load. In some embodiments, Tris is at a final concentration from 30 to 80 mM such as 50 mM in the load. In some embodiments, the pH of the load is from 7.0 to 9.2 such as 7.5. In some embodiments, the loading buffer further comprises zinc or a salt thereof preferably zinc chloride. In some embodiments, the concentration of zinc or salt thereof preferably zinc chloride in the load is equal to, two times, three times or four times the molar concentration of the polypeptide containing an Fc region in the load.

In some embodiments, hydrophobic interaction chromatography is conducted in flow-through mode and comprises a step of collection of the flow-through. In some embodiments, hydrophobic interaction chromatography is conducted in bind-elute mode and comprises an elution step with one or more elution buffers.

At the end of the hydrophobic interaction chromatography step described herein above, a hydrophobic interaction chromatography eluate or flow-through comprising the Fc region containing polypeptide is obtained.

### Method of purifying a polypeptide containing an Fc region - viral filtration, UF/DF, formulation and drug substance storage

In some embodiments, after the hydrophobic interaction chromatography step, the eluate or flow-through comprising the Fc region containing polypeptide is optionally subjected to a viral filtration step. At the end of the virus filtration step, a virus filtered composition comprising the Fc region containing polypeptide is obtained.

In some embodiments, the virus filtered composition comprising the Fc region containing polypeptide is optionally subjected to an ultrafiltration/diafiltration step. According to an embodiment of the invention, the ultrafiltration step can be a direct flow filtration (DFF) or tangential flow filtration step (TFF). In some embodiments, the ultrafiltration step is tangential flow filtration step (TFF).

In some embodiments, the ultrafiltration and diafiltration step is followed by formulation of the Fc region containing polypeptide in a formulation buffer. In some embodiments, the formulation buffer comprises at least two amino acids and a sugar. In some embodiment, the formulation buffer comprises at least two amino acids, a sugar, a salt, a surfactant and a buffer. In some embodiments the formulation comprises L-arginine, L-methionine, trehalose, sodium chloride, polysorbate 20 and acetic acid. In some embodiments, L-arginine is at a concentration from 10 to 50 mM, 20 to 40 mM such as 30 mM. In some embodiments, methionine is at a concentration from 1.0 to 10mM, 2.0 to 8.0 mM such as 5 mM. In some embodiments, trehalose is at a concentration from 100 to 300 mM, 150 to 300 mM, 200 to 300 mM such as 250 mM. In some embodiments, sodium chloride is at a concentration from 20 to 70 mM such as 50 mM. In some embodiments, polysorbate 20 is at a concentration from 0.005% (w/v) to 0.2% (w/v), 0.01% (w/v) to 0.05% (w/v) such as 0.02% (w/v). In some embodiments, acetic acid is at a concentration from 1.0 to 15 mM, 2.0 to 12 mM such as 10 mM at pH 5.8. In some embodiments, the formulation comprises 20 to 60 g/L of Fc region containing polypeptide. In some embodiments, the formulation comprises 30 to 50 g/L of Fc region containing polypeptide. In some embodiments, the formulation comprises 40 g/L of Fc region containing polypeptide.

In some embodiments, the Fc region containing polypeptide in the formulation buffer is filtered and the drug substance is stored preferably under freezing conditions or the drug product preferably at 2 to 8 °C.

### Method of purifying a polypeptide containing an Fc region - after Protein A chromatography

In some embodiments, the Protein A eluate comprising the Fc region containing polypeptide from the Protein A chromatography step as described in that section is subjected to one or more further purification steps.

In some embodiments, the Protein A eluate comprising the Fc region containing polypeptide from the Protein A chromatography step as described in in that section is subjected to a viral inactivation step, at least one filtration step, and at least one polishing step. In some embodiments, the polishing step is selected from one or more of ion exchange chromatography and hydrophobic interaction chromatography.

In some embodiments, the Protein A eluate comprising the Fc region containing polypeptide from the Protein A chromatography step as described in that section is subjected to one or more further purification steps comprising:
(i) viral inactivation,
(ii) a first UF/DF,
(iii) anion exchange chromatography preferably in bind-elute mode,
(iv) hydrophobic interaction chromatography preferably in flow-through mode,
(v) viral filtration,
(vi) a second UF/DF,
(vii) formulation,
(viii) drug substance storage.

In some embodiments, the Protein A eluate comprising the Fc region containing polypeptide from the Protein A chromatography step as described in in that section is subjected to the following sequential steps:
(i) viral inactivation,
(ii) a first UF/DF,
(iii) anion exchange chromatography preferably in bind-elute mode,
(iv) hydrophobic interaction chromatography preferably in flow-through mode,
(v) viral filtration,
(vi) a second UF/DF,
(vii) formulation,
(viii) drug substance storage.

The embodiments for the steps above, are described in the previous sections.

### Recovery of the Fc region containing polypeptide Protein A chromatography step

It has been found that the method of Protein A chromatography step as described herein provides improved recovery. In some embodiments, the method of Protein A chromatography step as described herein provides improved recovery.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer greater than 3.5, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer greater than 3.5, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 M to 2.5 M L-arginine HCL in the elution buffer, (ii) pH of the elution buffer greater than 3.5, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 M to 2.5 M L-arginine HCL in the elution buffer, (ii) pH of the elution buffer greater than 3.5, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 M to 2.5 M L-arginine HCL in the elution buffer, (ii) pH of the elution buffer greater than 3.5, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 M to 2.5 M L-arginine HCL in the elution buffer, (ii) pH of the elution buffer greater than 3.5, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.8 M to 2.3 M such as 2.0 M L-arginine HCL in the elution buffer, (ii) pH of the elution buffer greater than 3.5, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.8 M to 2.3 M such as 2.0 M L-arginine HCL in the elution buffer, (ii) pH of the elution buffer greater than 3.5, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer greater than 4.0, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer greater than 4.0, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 M to 2.5 M L-arginine HCL in the elution buffer, (ii) pH of the elution buffer greater than 4.0, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 M to 2.5 M L-arginine HCL in the elution buffer, (ii) pH of the elution buffer greater than 4.0, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 M to 2.5 M L-arginine HCL in the elution buffer, (ii) pH of the elution buffer greater than 4.0, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 M to 2.5 M L-arginine HCL in the elution buffer, (ii) pH of the elution buffer greater than 4.0, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.8 M to 2.3 M such as 2.0 M L-arginine HCL in the elution buffer, (ii) pH of the elution buffer greater than 4.0, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.8 M to 2.3 M such as 2.0 M L-arginine HCL in the elution buffer, (ii) pH of the elution buffer greater than 4.0, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer is 4.3, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer is 4.3, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 M to 2.5 M L-arginine HCL in the elution buffer, (ii) pH of the elution buffer is 4.3, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 M to 2.5 M L-arginine HCL in the elution buffer, (ii) pH of the elution buffer is 4.3, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 M to 2.5 M L-arginine HCL in the elution buffer, (ii) pH of the elution buffer is 4.3, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 M to 2.5 M L-arginine HCL in the elution buffer, (ii) pH of the elution buffer is 4.3, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.8 M to 2.3 M such as 2.0 M L-arginine HCL in the elution buffer, (ii) pH of the elution buffer is 4.3, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.8 M to 2.3 M such as 2.0 M L-arginine HCL in the elution buffer, (ii) pH of the elution buffer is 4.3, and (iii) the Fc region being a variant of the human IgG1, IgG2, or IgG4 subclass and comprising amino acid substitutions M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 2.0 M L-arginine HCL in the elution buffer, (ii) pH of the elution buffer is 4.3, and (iii) the Fc region being a variant of the human IgG1, IgG2, or IgG4 subclass and comprising at least one amino acid substitution selected from M252Y, S254T, and T256E according to EU numbering.

In some embodiments, a method of purifying a polypeptide containing an Fc region is provided, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 2.0 M L-arginine HCL in the elution buffer, (ii) pH of the elution buffer is 4.3, and (iii) the Fc region being a variant of the human IgG1, IgG2, or IgG4 subclass and comprising amino acid substitutions M252Y, S254T, and T256E according to EU numbering.

The invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

Thus, the invention provides a method of purifying a polypeptide containing an Fc region comprising a Protein A chromatography step, wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

### Method for prevention of aggregates formation when purifying a polypeptide containing an Fc region

It has been found that the method of Protein A chromatography step as described herein is for prevention of formation of aggregates. In some embodiments, the method of Protein A chromatography step as described herein prevents formation of aggregates as measured by size exclusion chromatography.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 3.5, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 3.5, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 0.5 M to 2.5 M L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution (s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.5 M to 2.5 M L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 4.0, wherein the Fc region is a variant of the human IgG1, IgG2, or IgG4 subclass and comprises at least one amino acid substitution(s) selected from M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising at least one amino acid substitution(s) selected from M252Y, S254T, and T256E according to EU numbering and subjected to a Protein A chromatography step comprising an elution step with an elution buffer in which the elution buffer does not comprise L-arginine HCL and has a pH of 3.5 or less.

The invention provides a method for prevention of aggregates formation when purifying a polypeptide containing an Fc region of the human IgG1, IgG2, or IgG4 subclass, the method comprising,
- providing a variant of the Fc region, wherein the variant comprises at least one or all amino acid substitutions selected from M252Y, S254T, and T256E according to EU numbering,
- a Protein A chromatography step,
wherein the Protein A chromatography step comprises an elution step with an elution buffer to obtain a Protein A eluate, wherein the elution buffer comprises 1.8 M to 2.3 M such as 2.0 M L-arginine HCL, and wherein the elution buffer has a pH of higher than 4.0, wherein the Fc region is a variant of the human IgG1, IgG2, or IgG4 subclass and comprises amino acid substitutions M252Y, S254T, and T256E according to EU numbering. In this context, the recovery of the Fc region containing polypeptide after the Protein A chromatography step is higher and the percentage of high molecular weight aggregates is lower as compared to the same Fc region containing polypeptide not comprising amino acid substitutions M252Y, S254T, and T256E according to EU numbering and subjected to a Protein A chromatography step comprising an elution step with an elution buffer in which the elution buffer does not comprise L-arginine HCL and has a pH of 3.5 or less.

### Examples

### Example 1- Purification of a polypeptide containing an Fc region

In this example, Fc region containing polypeptides, ACE2-IgG1 and ACE2-IgG4 were purified by the following sequential steps:
(i) Protein A chromatography
(ii) Virus inactivation
(iii) 1^{st} UF/DF
(iv) Anion Exchange Chromatography in bind-elute mode
(v) Hydrophobic Interaction Chromatography in Flow-through mode
(vi) Virus Filtration
(vii) 2^{nd} UF/DF
(viii) Formulation
(ix) Drug substance storage

### Protein A Chromatography

The Protein A chromatography is performed as a capture step in order to remove most of the impurities coming from the sterile filtered fermentation broth. Operating parameters of the Protein A chromatography can be found in Table 1, below.

**Table 1 - Protein A chromatography**

| **Protein A Chromatography** | |
|---|---|
| General Set-up | Resin: Amsphere A3 |
| | Column: Repligen |
| | Column ID: 45.7 cm |
| | Bed height: 20.2 cm |
| | Column volume: 33.13 L |
| | Binding mode: bind-elute |
| | Flow direction: downflow |
| Equilibration | Buffer, 40 mM MES, 150 mM NaCl, pH:6.5 (Wash 1) |
| | Volume, 4 CV |
| | Flow, 492 L/h |
| Load of filtered supernatant | Flow, 397 L/h |
| | Max Load: ≤ 50 g/L |
| Intermediate Wash | Wash 1 |
| | Buffer: 40 mM MES, 150 mM NaCl, pH:6.5 |
| | Volume: 4 CV |
| | Flow: 497 L/h |
| | Wash 2 |
| | Buffer: 40 mM MES, 1 M NaCl, pH:6.5 |
| | Volume: 2 CV |
| | Flow: 497 L/h |
| | Wash 3 |
| | Buffer: 40 mM MES, 50 mM NaCl, pH:6.5 |
| | Volume: 2 CV |
| | Flow: 497 L/h |
| | Wash 4 |
| | Buffer: 40 mM Na-acetate, 50 mM NaCl, pH:5.6 |
| | Volume: 3 CV |
| | Flow: 497 L/h |
| Elution | Buffer: 40 mM Na-acetate,50 mM NaCl, 2 M Arg, pH:4.3 |
| | Volume: 3 CV |
| | Frac. Col., from 100 mAU to 200 mAU |
| | Flow: 100 L/h |

### Virus inactivation

Virus inactivation step is carried out for about 60 min in order to reduce potential virus contamination as a part of virus removal procedure. Operating parameters of the virus inactivation can be found in Table 2.

**Table 2 - Virus inactivation**

| **Virus Inactivation** | |
|---|---|
| | |
| Protein A eluate: caprylic acid addition and pH adjustment for virus inactivation | Adjust pH 5.5-5.6 with 200 mM MES hydrate, 50 mM NaCl, pH:6.2 and/or 0.5 M acetic acid, 50 mM NaCl and/or 1 M Tris, 50 mM NaCl pH:9.0 |
| | Adjust final caprylate concentration to 25 mM with 250 mM sodium caprylate, 50 mM NaCl or with 40 mM Na-acetate, 50 mM NaCl, 10% caprylic acid, pH:5.6 |
| | Target pH: 5.5-5.7 |
| | Mixing speed: 70 rpm |
| Incubation | Time: 60-80 minutes |
| pH adjustment after virus inactivation | Buffer: 1 M Tris, 50 mM NaCl pH:9.0 |
| | Mixing speed: 70 rpm |
| | Target pH: 7.4-7.6 |

High molecular weight aggregates were measured by size exclusion chromatography as described in Example 4. The following results show that the method restricts the aggregates formation in this step to less than 1%:
- %HMW measured after Protein A chromatography: 11.7%
- %HMW measured after approximately 60 minutes of virus inactivation: 12.1%.

### 1^{st} Ultrafiltration/Diafiltration (UF/DF)

After virus inactivation an UF/DF step is performed in order to change the buffer environment of the molecule. The product is concentrated to 10 g/L and after that diafiltration is carried out with at least 7 diafiltration volumes by using AEX equilibration buffer: 50 mM Tris-HCl, 50 mM NaCl, pH 7.5. Operating parameters of the 1^{st} UF/DF step can be found in Table 3

**Table 3 - 1^{st} UF/DF**

| **1^{st} UF/DF** | |
|---|---|
| General Set-up | Pellicon 3 cassettes 30 kDa, 1.14 m2: 3 pcs |
| Concentration | Target concentration: ≤ 14 g/l |
| | Target TMP: 1.0±0.1 bar |
| | Feed flow rate: ~ 880 L/h/m2 (based on calculation) |
| | Permeate flow rate: ~150 L/h (based on observation) |
| | Concentration starts once 2nd cycle of virus inactivated liquid is collected |
| Buffer exchange | Buffer: 50 mM Tris, 50 mM NaCl pH:7.5 |
| | Buffer exchange volume: 7x |
| | Target TMP: 1.0±0.1 bar |
| | Feed flow rate: ~ 1080 L/h/m2 (based on calculation) |
| | Permeate flow rate: ~310 L/h (based on observation) |

### Anion Exchange Chromatography

After first UF/DF step anion exchange chromatography is performed by using a linear gradient formation. Eluate is collected in different fractions and analysed for aggregation and charge variant levels (in-process control). Based on acceptance criteria pooling of the fractions is performed. Operating parameters of the AEX can be found in Table 4.

**Table 4 - Anion Exchange Chromatography**

| **Anion Exchange Chromatography** | |
|---|---|
| General Set-up | Resin: Capto Q |
| | Column: packed in-house |
| | Column ID: 60 cm |
| | Bed height: 15 cm |
| | Column volume: 42.41 L |
| | Binding mode: bind-elute |
| Equilibration | Buffer. WFI |
| | Volume: 3CV |
| | Flow: 424L/h - upflow |
| | Buffer: 2 M NaCl |
| | Volume: 3CV |
| | Flow: 424 L/h - upflow |
| | Buffer: 50 mM Tris, 50 mM NaCl pH:7.5 |
| | Volume: 3CV |
| | Flow: 424 L/h - downflow |
| Load | Flow: 424 L/h - downflow |
| | Max Load: ≤ 25 g/L |
| Intermediate Wash | Buffer: 50 mM Tris, 50 mM NaCl pH:7.5 |
| | Volume: 2 CV |
| | Flow: 424 L/h - downflow |
| Elution | Buffer A: 50 mM Tris, 50 mM NaCl pH:7.5 |
| | Buffer B: 50 mM Tris, 500 mM NaCl pH:7.5 |
| | Gradient: 0%-80% B |
| | Slope of gradient: 5% B/CV |
| | Flow: 424 L/h - downflow |
| | Start of fraction collection: 0.1 AU |

### Hydrophobic Interaction Chromatography

In the following step a hydrophobic interaction chromatography is performed in flowthrough mode. This step is a polishing step in the overall cleaning procedure in order to remove the remaining process and product related impurities and aggregates. The flowthrough product is collected based on UV280 signal. Operating parameters of HIC step in flow-through mode can be found in Table 5.

**Table 5 - Hydrophobic interaction chromatography**

| **Hydrophobic Interaction Chromatography Flow-through mode** | |
|---|---|
| Adjustment of eluate from AEX | Target: 50 mM Tris, 560 mM (NH4)2SO4, pH:7.5; (NH4)2SO4 concentration adjusted with stock solution: 50 mM Tris, 3 M (NH4)2SO4, pH:7.5 |
| General Set-up | Resin: Capto Phenyl |
| | Column: Cytiva |
| | Column ID: 35.9 cm |
| | Bed height: 20 cm |
| | Column volume: 20.24 L |
| | Binding mode: Flow-through mode |
| | Flow direction: downflow |
| Equilibration | BufferA: 50 mM Tris, 560 mM (NH4)2SO4, pH:7.5 |
| | Volume: 3 CV |
| | Flow: 152 L/h |
| Load | Flow 152 L/h |
| | Max Load: ≤ 25 g/L |
| Elution/Collection | BufferA: 50 mM Tris, 560 mM (NH4)2SO4, pH:7.5 |
| | Flow 152 L/h |
| | Start of the fraction collection: 0.05 AU |
| | End of fraction collection: < 0.1 AU |

An alternative method of hydrophobic chromatography step in flow-through mode includes replacing the ammonium buffer in the table above with a buffer comprising 50 mM Tris, 150 mM NaCl pH 7.5.

Hydrophobic interaction chromatography can also be performed in bind-elute mode. Operating parameters of HIC step in bind-elute mode can be found in Table 6.

**Table 6 - Hydrophobic interaction chromatography**

| **Hydrophobic Interaction Chromatography Bind-elute mode** | |
|---|---|
| Adjustment of eluate from AEX | Target: 50 mM Tris, 660 mM (NH4)2SO4, pH:7.5; (NH4)2SO4 concentration adjusted with stock solution: 50 mM Tris, 3 M (NH4)2SO4, pH:7.5 |
| General Set-up | Resin: Capto Phenyl |
| | Column: Cytiva |
| | Column ID: 35.9 cm |
| | Bed height: 20 cm |
| | Column volume: 20.24 L |
| | Binding mode: Bind-elute |
| | Flow direction: downflow |
| Equilibration | BufferA: 50 mM Tris, 660 mM (NH4)2SO4, pH:7.5 |
| | Volume: 3 CV |
| | Flow: 152 L/h |
| Load/Elution | Flow: 152 L/h |
| | Max Load: ≤ 25 g/L |
| | Loading conductivity: ~110 mS/cm |
| | After loading flush with 1CV 0%B, |
| | Buffer B: WFI |
| | Gradient: 0 - 52% B in 10 CV plus 5 CV at 52 %B |
| | Start of the fraction collection: 0.05 AU |
| | End of fraction collection: < 0.1 AU |

### Virus Filtration

The virus filtration step is an orthogonal method to remove viruses from product stream during DSP technology. Operating parameters of the virus filtration can be found in Table 7.

**Table 7 - Virus Filtration**

| **Virus Filtration** | |
|---|---|
| Filter configuration | Device: PLANOVA BIOEX |
| | Filtration area: 4 m2 |
| | Amount of filter units: 1 pc/ cycle of HIC |
| | Before Virus Filter a prefilter was used (Sartorius Virosart Max 30") |
| | Filtration area: 2,1 m2 |
| | Amount of filter units, 1 pc for each virus filter |
| Pre Flush | Buffer: 50mM Tris, 150 mM NaCl, pH:7,5 |
| | Volume: 60 kg (12+48 for wetting and equilibration, respectively) |
| | Pressure: 0.3 bar |
| | Temperature: ambient |
| Filtration | Max. Volume: 128 kg/m2 (executed 64 kg/m2) |
| | Pressure: 2 ±0.2 bar |
| | Temperature: ambient |
| Post Flush | Buffer: 50mM Tris, 150 mM NaCl, pH:7,5 |
| | Volume: 40 kg |
| | Pressure: 2 ±0.2 bar (executed: 1.65-1.84 bar) |
| | Temperature: ambient |

### 2^{nd} Ultrafiltration/Diafiltration (UF/DF) step

After virus filtration 2^{nd} UF/DF step is performed in order to set-up the final concentration of the product and to change the buffer environment close to the final formulation buffer except
some excipients which are not compatible with ultrafiltration. Operating parameters of the 2^{nd} UF/DF step can be found in Table 8.

**Table 8 - Ultrafiltration/Diafiltration**

| **Final Ultrafiltration/Diafiltration** | |
|---|---|
| Ultra Filter | Device, Millipore Pellicon 3 30 kDa cut off |
| | Filtration area, 3 times 1,14 m2 |
| Concentration 1. | 1st concentration is done during virus filtration |
| | Target ≤ 15 g/L, ≤ 50 L |
| | Permeate Flow: 100 L/h |
| | TMP: 0.2-0.4 bar |
| | Temperature: ambient |
| Diafiltration | Diafiltration volume: 8X+30kg |
| | Buffer: 30 mM Arginine, 250 mM Trehalose, 5 mM Metionine, 50 mM NaCl, 10 mM acetic acid pH:5.8 |
| | Permeate Flow: 135 L/h |
| | TMP: 0.2-0.4 bar |
| | Temperature: ambient |
| Concentration 2 | Adjust target concentration 65 g/L |
| | Permeate Flow: 90-100 L/h |
| | TMP: 0.2-0.4 bar |
| | Temperature: ambient |

### Formulation and Final Filtration

The purpose of the formulation is to adjust the final excipient composition and the final concentration of the drug substance (DS). In this step the product solution is diluted with formulation buffer that contains 0.1% Polysorbate 20. This is the stock solution which will be mixed to the concentrated product solution in order to achieve the final protein concentration of the bulk drug substance at 40 g/L with the following formulation buffer composition (the protein concentration is adjusted to 40 g/L if needed using the formulation buffer):
- 0.02% (w/v) Polysorbate 20
- 30 mM L-Arginine
- 250 mM Trehalose
- 5 mM L-Methionine
- 50 mM NaCl
- 10 mM Acetic acid
- pH 5.8

This final drug substance with the above-mentioned buffer composition is filtered with 0.2 µm filter.

### Storage

The drug substance is stored and frozen at -80 °C as final storage temperature.

### Example 3 - Addition of Zinc in the loading buffer or equilibration buffer in Hydrophobic Interaction Chromatography step

The ACE2-IgG protein harbors two zinc binding sites, in theory a stoichiometry of 1:2 (protein: zinc) is expected in case the protein is fully occupied with zinc. A loss in protein-bound zinc was observed within the protein purification process sequence and via addition of zinc to the load material or to the equilibration buffer within the HIC process step, the protein-bound zinc content was increased successfully as analysed with inductively coupled plasma mass spectrometry as shown in Table 9 below.

**Table 9 - Zinc addition**

| | Fc region containing polypeptide: Zinc |
|---|---|
| After Protein A chromatography step | 1:1.59 |
| After first UF/DF | 1:1.32 |
| After anion exchange chromatography | 1:1.44 |
| After hydrophobic chromatography | Without Zinc addition (control run): 1:1.49 |
| | With Zinc addition to the equilibration/ running buffer: 1:1.60 |
| | With Zinc addition to the load: 1:1.65 |

Zinc stabilizes the structure of the protein with less fluctuations being expected with a fully occupied protein. It is expected that the Fc-containing polypeptide would be more stable under this method.

### Example 4 - Optimization of Protein A chromatography

The Protein A Chromatography step was optimized for highest possible recovery and prevention of formation of aggregates. In a first set of experiments, ACE2-IgG fusion protein (which is equally applicable to ACE2-IgG1 and ACE2-IgG4) was eluted in a Protein A chromatography step under three elution conditions as follows:
**Condition 1:** 40 mM Acetate, 50 mM NaCl + 10% Mannitol at pH 3.5
**Condition 2:** 40 mM Acetate, 50 mM NaCl + 2000mM L-Arginine HCL at pH 3.5
**Condition 3:** 40 mM Acetate, 50 mM NaCl + 2000 mM L-Arginine HCL at pH 4.3

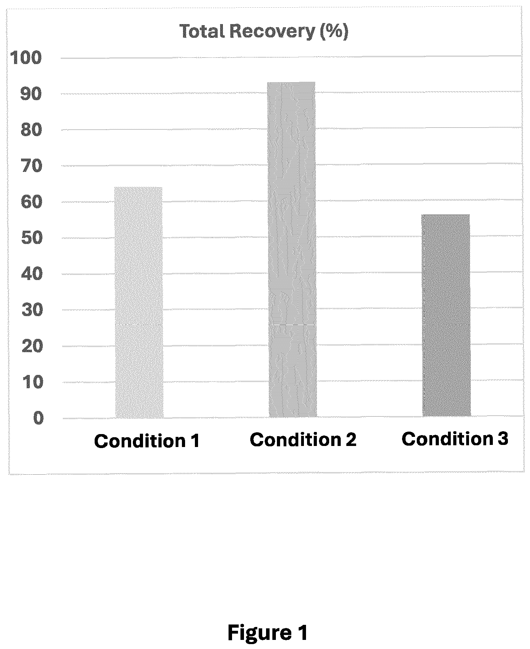

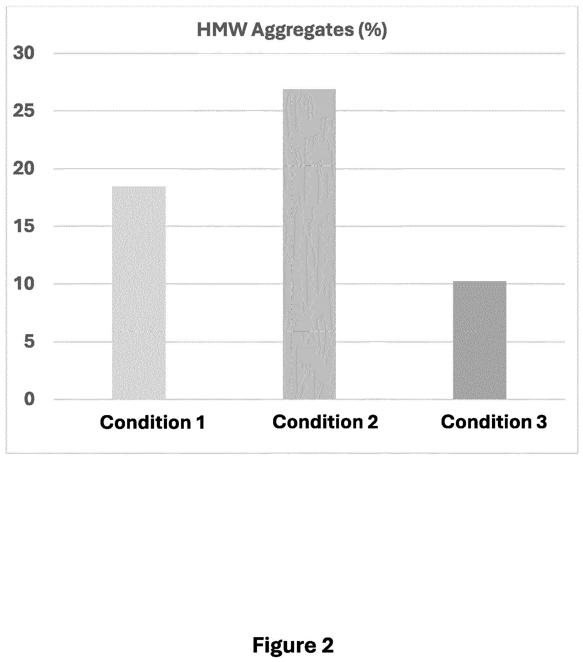

The results of total recovery and high molecular weight (HMW) species (aggregates) are shown in Figure 1 and Figure 2 and in Table 10 below.

**Table 10 - Total recovery (%) and aggregates (HMW %)**

| **Conditions** | **Total recovery (%)** | **Aggregates (HMW%)** |
|---|---|---|
| Condition 1 | 64 | 18.45% |
| Condition 2 | 93 | 26.90% |
| Condition 3 | 56 | 10.25% |

Condition 2 (Arginine and low pH) was although good for total recovery, it led to highest percentage of aggregates. The lowest aggregates were achieved under condition 3 (Arginine and high pH).

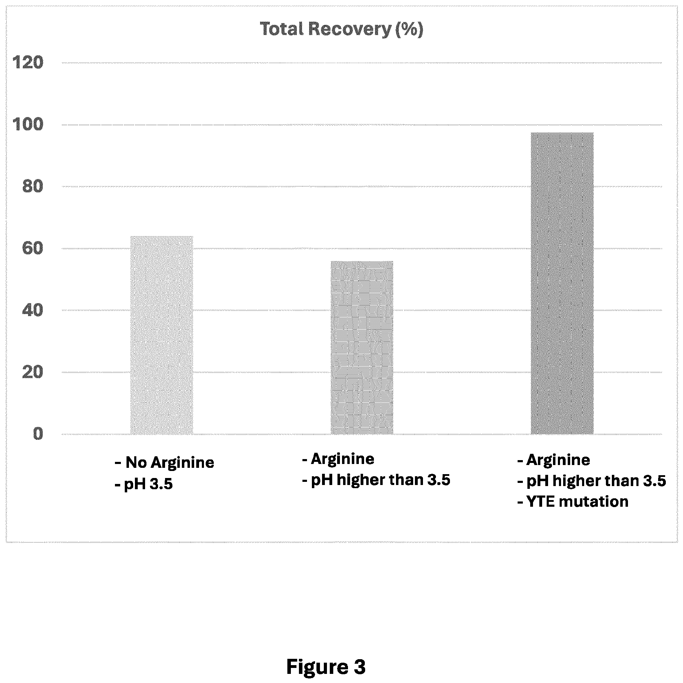

For further improving total recovery, the Fc region of the ACE2-IgG was mutated with amino acid substitutions M252Y, S254T, and T256E (YTE). Surprisingly, the results showed a synergistic effect of having 2M arginine at pH 4.3 in the elution buffer and the YTE mutation in the Fc region of the ACE2-IgG (see Figure 3 and Table 11, below).

**Table 11 - Total recovery (%)**

| **Conditions** | **Total recovery (%)** |
|---|---|
| No Arginine and pH 3.5 | 64 |
| Arginine at pH 4.3 | 56 |
| Arginine at pH 4.3 and YTE mutation | 97.4% |

Protein concentration is measured with analytical Protein A chromatography (filtered cell supernatant) and slope spectroscopy (eluate). The recovery is the protein amount after the purification step divided by the protein amount before the purification step multiplied with 100%. Aggregates are analysed with analytical size exclusion chromatography (SEC).

Slope spectroscopy measures the concentration of undiluted protein samples at different pathlengths. The absorbance values are measured at several pathlengths to create a section curve of the absorbances plotted against the pathlengths. The concentration is directly proportional to the slope in the linear region of the curve according to the Beer-Lambert's law, based on the sample extinction coefficient. The measurement was performed at 280 nm and at 320-350 nm to correct light scattering within the sample.

Analytical Protein A chromatography is performed as follows.
Solvents:
   - Mobile phase A: 50 mM sodium phosphate + 400 mM NaCl, pH 7.4 (binding buffer)
   - Mobile phase B: 50 mM Glycine-HCl + 150 mM NaCl, pH 2.5 (elution solvent)
   - Seal wash: Acetonitrile / MilliQ water (5:95)
   - Purge and needle wash: MilliQ water
Sample preparation:
   - SST sample in a concentration of 0.20 mg/mL from the antibody/fusion protein reference standard was created and diluted if necessary in mobile phase A
   - Calibration curve was created with several standards between 0.20 and 5.0 mg/mL from the antibody/fusion protein reference standard; standard solution was diluted in mobile phase A if necessary
   - Two analytical control samples were prepared at concentrations of 0.4 and 4.0 mg/mL
   - A blank was prepared from mobile phase A
   - If titres < 5 mg/mL were expected no dilution was required. If titres > 5 mg/mL were expected, the samples were diluted with mobile phase A to be within the range of 0.2 - 5 mg/mL
Instrumental parameters:
   - System: Acquity H-class bio equipped with TUV detector
   - Column: POROS A-20 µm, 2.1 mm X 30 mm
   - Column oven temperature: 30⁰C
   - Sample manager temperature: 7⁰C
   - Injection volume (µL): 10
   - UV detection, single wavelength at 280 nm, sampling rate: 10 points/sec
   - Integration settings Empower: ApexTrack, Integration between 1.6 to 1.9 min, liftoff: 0.05%, peak width: 2.8 sec, Detection threshold: 5000.
Data analysis:
   Calibration curve and analytical control samples:
   A calibration curve was created with the antibody/fusion protein reference standards between 0.2 mg/mL and 5.0 mg/mL by plotting peak areas against concentration. The correlation coefficient of the calibration curve was checked and was R2>0.99. The concentrations of the analytical control standards at 0.4 mg/mL and 4 mg/mL were calculated and the accuracy of the results was checked with the theoretical values. Back-calculated concentrations were within 93-107 % of the theoretical value.
Sample analysis:
   The concentration of unknown analytical samples was calculated using the calibration curve.

Size exclusion chromatography was performed as follows.
Solvents:
   - Mobile phase A: 20 mM Sodium phosphate, 150 mM sodium chloride, pH 7.0
   - Seal Wash: MilliQ:Acetonitrile (95:5)
   - Purge and needle wash: MilliQ
Sample preparation:
   - Sample concentration: about 1 mg/mL
   - BEH200 SEC standard: complete vial contents dissolved in 1.0 mL of mobile phase A
Instrumental parameters:
   - System: Acquity H-class+ bio, equipped with extended column oven, TUV detector
   - 15 µg injection (injection volume was adjusted based on concentration)
   - Column: ACQUITY UPLC Protein BEH SEC Column, 200Å, 1.7 µm, 4.6 mm X 150 mm, 10K - 500K
   - Guard column: ACQUITY UPLC Protein BEH SEC Guard Column, 200Å, 1.7 µm, 4.6 mm X 30 mm, 10K - 500K
   - Column temperature: 30 °C
   - Mobile phase: 20 mM NaPO4, 150 mM NaCl, pH 7.0
   - Gradient: Isocratic run
   - Run time: 10 minutes
   - Flow rate: 0.3 mL/min
   - UV detection: dual wavelength, 214 and 280 nm, sampling rate: 2 points/sec
   - After analysis: flush column with MilliQ and subsequently with 10% methanol
   - Integration settings Empower: ApexTrack, integration between 2 to 6.5 min, touchdown: 0.05%, minimum height: 500.

### List of Items of the Invention

**1.** A method of purifying a polypeptide containing an Fc region, the method comprising
   - a Protein A chromatography step, wherein the Protein A chromatography step comprises an elution step with an elution buffer to obtain a Protein A eluate, wherein the elution buffer comprises arginine, and wherein the elution buffer has a pH of higher than 3.5,
   wherein the Fc region is a variant of the human IgG1, IgG2, or IgG4 subclass and comprises at least one amino acid substitution selected from M252Y, S254T, and T256E according to EU numbering.
**2.** The method of item 1, wherein the Fc region comprises amino acid substitutions M252Y, S254T, and T256E according to EU numbering.
**3.** The method of item 1 or 2, wherein the elution buffer has a pH from 3.8 or higher.
**4.** The method of any one of items 1 to 3, wherein the elution buffer has a pH of 4.0 or higher such as from 4.2 to 4.5 such as 4.3.
**5.** The method of any one of items 1 to 4, wherein the elution buffer comprises 0.5 to 2.5 M L-arginine HCL, such as 1.0 to 2.5 M, 1.5 to 2.5 M, 1.8 to 2.3 M L-arginine HCL such as 2.0 M L-arginine HCL.
**6.** The method of any one of items 1 to 5, wherein the method comprises a virus inactivation step on the Protein A eluate comprising adding caprylic acid or a salt thereof.
**7.** The method of any one of items 1 to 6, wherein the method comprises a virus inactivation step on the Protein A eluate comprising the following sequential steps,
   (i) pH adjustment comprising adjusting the pH to greater than 5.0 such as from 5.2 to 6.0 such as 5.6,
   (ii) adding caprylic acid or a salt thereof.
**8.** The method of item 7, wherein the method comprises adding sodium caprylate and the final concentration of sodium caprylate is from 10 to 30 mM.
**9.** The method of any one of items 6 to 8, wherein after the virus inactivation step, the method comprises one or more further purification steps.
**10.** The method of any one of items 6 to 9, wherein after the virus inactivation step, the method comprises the following sequential steps:
   (i) a first UF/DF,
   (ii) anion exchange chromatography preferably in bind-elute mode,
   (iii) hydrophobic interaction chromatography preferably in flow-through mode,
   (iv) viral filtration,
   (v) a second UF/DF,
   (vi) formulation,
   (vii) drug substance storage.
**11.** The method of item 10, wherein hydrophobic interaction chromatography comprises a loading step comprising loading a load onto the hydrophobic interaction chromatography column, wherein the load comprises eluate obtained from the anion exchange chromatography comprising the polypeptide containing an Fc region and a loading buffer, wherein the loading buffer comprises zinc or a salt thereof.
**12.** The method of item 11, wherein hydrophobic interaction chromatography comprises an equilibration step with an equilibration buffer comprising zinc or a salt thereof before the loading step.
**13.** The method of item 11 or 12, wherein the concentration of zinc or salt thereof in the equilibration buffer is two times or three times the molar concentration of the polypeptide containing an Fc region in the load.
**14.** The method of item 11 to 13, wherein the concentration of zinc or salt thereof in the loading buffer results in a concentration of the zinc or salt thereof in the load that is two times or three times the molar concentration of the polypeptide containing an Fc region in the load.
**15.** The method of item 11 to 14, wherein the concentration of zinc or salt thereof in the load is two times or three times the molar concentration of the polypeptide containing an Fc region in the load.
**16.** The method of any one of items 11 to 15, wherein the equilibration buffer, the loading buffer and/or the load comprises zinc chloride.
**17.** The method of any one of items 1 to 16, wherein the recovery of the Fc region containing polypeptide after the Protein A chromatography step is higher and the percentage of high molecular weight aggregates is lower as compared to the same Fc region containing polypeptide not comprising at least one or all amino acid substitution(s) selected from M252Y, S254T, and T256E according to EU numbering and subjected to a Protein A chromatography step comprising an elution step with an elution buffer in which the elution buffer does not comprise arginine and has a pH of 3.5 or less.
**18.** The method of any one of items 1 to 16, wherein the recovery of the Fc region containing polypeptide after Protein A chromatography step is higher and the percentage of high molecular weight aggregates is lower as compared to the same Fc region containing polypeptide not comprising at least one or all amino acid substitution(s) selected from M252Y, S254T, and T256E according to EU numbering and subjected to a Protein A chromatography step comprising an elution step with an elution buffer in which the elution buffer does not comprise arginine and has a pH of 4.0 or less.
**19.** A method of purifying a polypeptide containing an Fc region, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) L-arginine HCL in the elution buffer, (ii) pH of the elution buffer being greater than 3.5, and (iii) the Fc region being a variant of the human IgG1, IgG2, or IgG4 subclass and comprising at least one amino acid substitution selected from M252Y, S254T, and T256E according to EU numbering.
**20.** A method of purifying a polypeptide containing an Fc region, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 0.5 to 2.5 M L-arginine HCL in the elution buffer, (ii) pH of the elution buffer being greater than 3.5, and (iii) the Fc region being a variant of the human IgG1, IgG2, or IgG4 subclass and comprising at least one amino acid substitution selected from M252Y, S254T, and T256E according to EU numbering.
**21.** A method of purifying a polypeptide containing an Fc region, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 1.5 to 2.5 M L-arginine HCL in the elution buffer, (ii) pH of the elution buffer being greater than 3.5, and (iii) the Fc region being a variant of the human IgG1, IgG2, or IgG4 subclass and comprising at least one amino acid substitution selected from M252Y, S254T, and T256E according to EU numbering.
**22.** A method of purifying a polypeptide containing an Fc region, the method comprising a Protein A chromatography step, wherein the Protein A chromatography step comprises an elution step with an elution buffer, and wherein the following three conditions provide a synergistic effect in improving total recovery of an Fc region containing polypeptide in a Protein A chromatography step: (i) 2.0 M L-arginine HCL in the elution buffer, (ii) pH of the elution buffer being 4.3, and (iii) the Fc region being a variant of the human IgG1, IgG2, or IgG4 subclass and comprising at least one amino acid substitution selected from M252Y, S254T, and T256E according to EU numbering.
**23.** A method for prevention of aggregates formation when purifying a polypeptide containing an Fc region of the human IgG1, IgG2, or IgG4 subclass, the method comprising,
   - providing a variant of the Fc region, wherein the variant comprises at least one or all amino acid substitutions selected from M252Y, S254T, and T256E according to EU numbering,
   - a Protein A chromatography step, wherein the Protein A chromatography step comprises an elution step with an elution buffer, wherein the elution buffer comprises arginine, and wherein the elution buffer has a pH of higher than 3.5.
**24.** A method for prevention of aggregates formation when purifying a polypeptide containing an Fc region of the human IgG1, IgG2, or IgG4 subclass, the method comprising,
   - providing a variant of the Fc region, wherein the variant comprises at least one or all amino acid substitutions selected from M252Y, S254T, and T256E according to EU numbering,
   - a Protein A chromatography step, wherein the Protein A chromatography step comprises an elution step with an elution buffer, wherein the elution buffer comprises arginine, and wherein the elution buffer has a pH of 4.0 or higher.
**25.** A method for prevention of aggregates formation when purifying a polypeptide containing an Fc region of the human IgG1, IgG2, or IgG4 subclass, the method comprising,
   - providing a variant of the Fc region, wherein the variant comprises at least one or all amino acid substitutions selected from M252Y, S254T, and T256E according to EU numbering,
   - a Protein A chromatography step, wherein the Protein A chromatography step comprises an elution step with an elution buffer, wherein the elution buffer comprises arginine, wherein the elution buffer has a pH of higher than 3.5 such as 4.0 or higher, and wherein the elution buffer comprises 0.5 to 2.5 M arginine, such as 1.8 to 2.3 M arginine such as 2.0 M arginine.
**26. A** method of viral inactivation of an eluate obtained from a Protein A chromatography step comprising an Fc region containing polypeptide, the method comprising
   - eluting the Fc region containing polypeptide in the Protein A chromatography step using an elution buffer, wherein the elution buffer comprises arginine, and wherein the elution buffer has a pH of higher than 3.5 such as 4.0 or higher,
   - adding sodium caprylate at a final concentration from 10 to 30 mM.
**27.** The method of any one of items 1 to 26, wherein the polypeptide is a fusion protein.
**28.** The method of any one of items 1 to 27, wherein the polypeptide has an isoelectric point of lower than 7 or lower than 6.
**29.** The method of any one of items 1 to 28, wherein the polypeptide is acid labile.
**30.** The method of any one of items 1 to 29, wherein the polypeptide is an IgG1 or IgG4 fusion protein.
**31.** The method of any one of items 1 to 30, wherein the polypeptide is an ACE2-IgG1 or ACE2-IgG4 fusion protein.

## Claims

1. A method of purifying a polypeptide containing an Fc region, the method comprising
- a Protein A chromatography step, wherein the Protein A chromatography step comprises an elution step with an elution buffer to obtain a Protein A eluate, wherein the elution buffer comprises arginine, and wherein the elution buffer has a pH of higher than 3.5,
wherein the Fc region is a variant of the human IgG1, IgG2, or IgG4 subclass and comprises at least one amino acid substitution selected from M252Y, S254T, and T256E according to EU numbering.

2. The method of claim 1, wherein the Fc region comprises amino acid substitutions M252Y, S254T, and T256E according to EU numbering.

3. The method of claim 1 or 2, wherein the elution buffer has a pH from 3.8 or higher.

4. The method of any one of claims 1 to 3, wherein the elution buffer has a pH of 4.0 or higher such as from 4.2 to 4.5 such as 4.3.

5. The method of any one of claims 1 to 4, wherein the elution buffer comprises 0.5 to 2.5 M L-arginine HCL, such as 1.0 to 2.5 M, 1.5 to 2.5 M, 1.8 to 2.3 M L-arginine HCL such as 2.0 M L-arginine HCL.

6. The method of any one of claims 1 to 5, wherein the method comprises a virus inactivation step on the Protein A eluate comprising adding caprylic acid or a salt thereof.

7. The method of any one of claims 1 to 6, wherein the method comprises a virus inactivation step on the Protein A eluate comprising the following sequential steps,
(i) pH adjustment comprising adjusting the pH to greater than 5.0 such as from 5.2 to 6.0 such as 5.6,
(ii) adding caprylic acid or a salt thereof.

8. The method of claim 7, wherein the method comprises adding sodium caprylate and the final concentration of sodium caprylate is from 10 to 30 mM.

9. The method of any one of claims 6 to 8, wherein after the virus inactivation step, the method comprises one or more further purification steps, preferably wherein after the virus inactivation step, the method comprises the following sequential steps:
(i) a first UF/DF,
(ii) anion exchange chromatography preferably in bind-elute mode,
(iii) hydrophobic interaction chromatography preferably in flow-through mode,
(iv) viral filtration,
(v) a second UF/DF,
(vi) formulation,
(vii) drug substance storage.

10. The method of claim 9, wherein hydrophobic interaction chromatography comprises a loading step comprising loading a load onto the hydrophobic interaction chromatography column, wherein the load comprises eluate obtained from the anion exchange chromatography comprising the polypeptide containing an Fc region and a loading buffer, wherein the loading buffer comprises zinc or a salt thereof preferably zinc chloride.

11. The method of claim 10, wherein hydrophobic interaction chromatography comprises an equilibration step with an equilibration buffer comprising zinc or a salt thereof preferably zinc chloride before the loading step.

12. The method of any one of claims 10 to 11, wherein the concentration of zinc or salt thereof preferably zinc chloride,
in the equilibration buffer is two times or three times the molar concentration of the polypeptide containing an Fc region in the load,
in the loading buffer results in a concentration of the zinc or salt thereof in the load that is two times or three times the molar concentration of the polypeptide containing an Fc region in the load, and/or
in the load is two times or three times the molar concentration of the polypeptide containing an Fc region in the load.

13. A method of viral inactivation of an eluate obtained from a Protein A chromatography step comprising an Fc region containing polypeptide, the method comprising
- eluting the Fc region containing polypeptide in the Protein A chromatography step using an elution buffer, wherein the elution buffer comprises arginine, and wherein the elution buffer has a pH of higher than 3.5 such as 4.0 or higher,
- adding sodium caprylate at a final concentration from 10 to 30 mM.

14. The method of any one of claims 1 to 13, wherein the polypeptide has an isoelectric point of lower than 7 or lower than 6.

15. The method of any one of claims 1 to 14, wherein the polypeptide is an IgG1 or IgG4 fusion protein, preferably wherein the polypeptide is an ACE2-IgG1 or ACE2-IgG4 fusion protein.
